# EUROPEAN PATENT APPLICATION

(11) **EP 4 364 733 A1**
(43) Date of publication of application: **08.05.2024**
(21) Application number: 22832934.8
(22) Date of filing: 21.06.2022
(51) Int. Cl.: A61K 31/277, A61P 35/00, A61P 39/02, A61P 39/04, C07C 291/10

(54) **COPPER CHELATOR, ANTICANCER AGENT AND PROPHYLACTIC OR THERAPEUTIC AGENT FOR WILSON'S DISEASE**

(30) Priority: 30.06.2021 JP 2021109058
(71) Applicant: Murata Manufacturing Co., Ltd., Nagaokakyo-shi, Kyoto 617-8555 (JP); National University Corporation Hokkaido University, Sapporo-shi, Hokkaido 060-0808 (JP)
(72) Inventor: TAKATA, Masachika, Nagaokakyo-shi, Kyoto 617-8555 (JP); SUNAHARA, Hirofumi, Nagaokakyo-shi, Kyoto 617-8555 (JP); WAKIMOTO, Toshiyuki, Sapporo-shi, Hokkaido 060-0808 (JP); MATSUDA, Kenichi, Sapporo-shi, Hokkaido 060-0808 (JP)
(74) Representative: Reeve, Nicholas Edward
(86) International application number: PCT/JP2022/024735
(87) International publication number: WO 2023/276786

(57) **Abstract**

A copper chelating agent contains at least one selected from the group consisting of a compound represented by the following Formula (1), a compound represented by the following Formula (2), a compound represented by the following Formula (3), a compound represented by the following Formula (4), a salt thereof, and a solvate thereof.

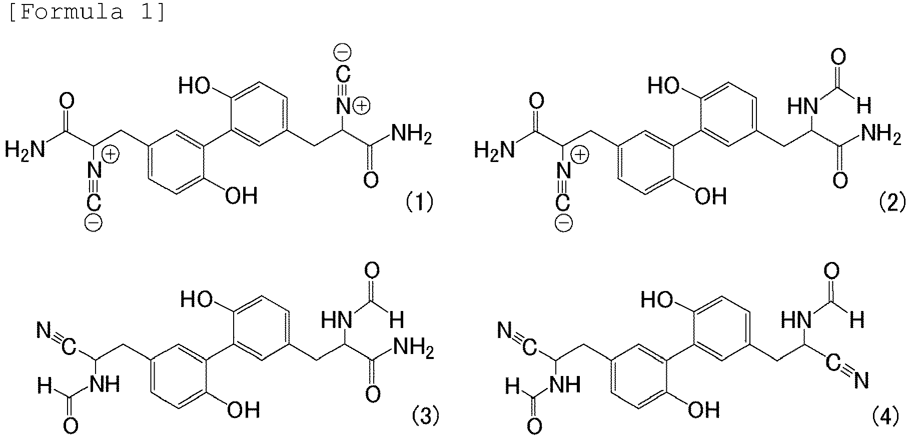

## Description

### TECHNICAL FIELD

The present invention relates to a copper chelating agent. The present invention also relates to an anticancer agent and a prophylactic or therapeutic agent for Wilson's disease.

### BACKGROUND ART

Physiologically active substances such as antibiotics have been hitherto found from metabolites of actinomycetes. Non-Patent Literature 1 describes that a P07101 strain of actinomycetes belonging to the genus Kitasatospora (Kitasatospora sp.) produced hazimycin A and analogous compounds thereof, and hazimycin A had an antimicrobial activity against gram-positive bacteria and Candida albicans.

### CITATION LIST

### - Non-Patent Literature

Non-Patent Literature 1: Acta Pharmaceutica Sinica B Volume 5, Issue 6, 2015, Pages 564 to 568

### SUMMARY OF INVENTION

### - Technical Problem

Incidentally, copper is an essential trace element in life, but when copper is excessively present in cells, it exhibits toxicity. As copper overload, Wilson's disease is known. In Wilson's disease, copper is deposited in organs of the whole body due to disorder of excretion of copper into bile, causing histological damage. Copper chelating agents are used for the treatment of Wilson's disease, but conventionally used therapeutic agents may have strong side effects. A substance having a copper chelating action is known to inhibit the action of cytochrome C oxidase, which is an enzyme essential for energy production of cells. When the activity of the cytochrome C oxidase is inhibited, not only energy is insufficient, but also toxic active oxygen is generated. It is considered that a substance having a copper chelating action exhibits, for example, an anticancer action by such an action (for example, Curr Med Chem. 2010; 17(25): pages 2685 to 2698). Therefore, a substance having a copper chelating action is also useful as an active ingredient of an anticancer agent. As described above, a compound having a copper chelating action is useful in, for example, the pharmaceutical field, and a new copper chelating agent is required.

Non-Patent Literature 1 describes that, as described above, hazimycin A had an antimicrobial activity against gram-positive bacteria and the like. However, the chelating action of hazimycin A and its analogous compounds has not been studied.

An object of the present invention is to provide a copper chelating agent. Another object of the present invention is to provide an anticancer agent and a prophylactic or therapeutic agent for Wilson's disease.

### - Solution to Problem

The copper chelating agent of the present invention contains at least one selected from the group consisting of a compound represented by the following Formula (1), a compound represented by the following Formula (2), a compound represented by the following Formula (3), a compound represented by the following Formula (4), a salt thereof, and a solvate thereof.

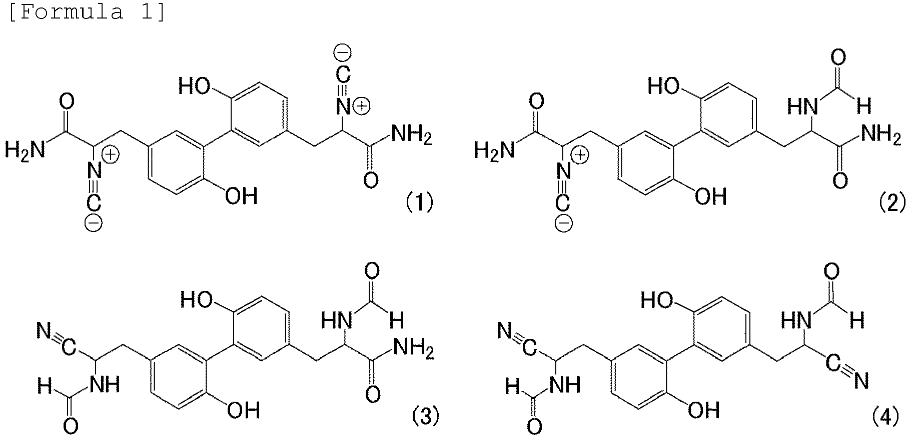

The anticancer agent of the present invention contains at least one selected from the group consisting of a compound represented by the above Formula (1), a compound represented by the above Formula (2), a compound represented by the above Formula (3), a compound represented by the above Formula (4), a salt thereof, and a solvate thereof.

The prophylactic or therapeutic agent for Wilson's disease of the present invention contains at least one selected from the group consisting of a compound represented by the above Formula (1), a compound represented by the above Formula (2), a compound represented by the above Formula (3), a compound represented by the above Formula (4), a salt thereof, and a solvate thereof.

### - Advantageous Effects of Invention

According to the present invention, a copper chelating agent can be provided. Further, according to the present invention, an anticancer agent and a prophylactic or therapeutic agent for Wilson's disease can be provided.

### BRIEF DESCRIPTION OF THE DRAWINGS

FIG. 1 shows the results of LC-MS analysis of a compound (1a) and a sample in which a copper ion is added to the compound (1a) (MS spectrum of ions at m/z 379.00) (A: the compound (1a), B: the sample in which a copper ion is added to the compound (1a)).
FIG. 2 shows the results of LC-MS analysis of a compound (1a) and a sample in which a copper ion is added to the compound (1a) (MS spectrum of ions at m/z 441.00) (A: the compound (1a), B: the sample in which a copper ion is added to the compound (1a)).
FIG. 3 shows an estimated chemical formula of a complex of a compound (1a) and a copper ion.
FIG. 4 shows the results of LC-MS analysis of a compound (2a) and a sample in which a copper ion is added to the compound (2a) (MS spectrum of ions at m/z 397.00) (A: the compound (2a), B: the sample in which a copper ion is added to the compound (2a)).
FIG. 5 shows the results of LC-MS analysis of a compound (2a) and a sample in which a copper ion is added to the compound (2a) (MS spectrum of ions at m/z 459.00) (A: the compound (2a), B: the sample in which a copper ion is added to the compound (2a)).
FIG. 6 shows an estimated chemical formula of a complex of a compound (2a) and a copper ion.

### DESCRIPTION OF EMBODIMENTS

Hereinafter, the copper chelating agent, the anticancer agent, and the prophylactic or therapeutic agent for Wilson's disease of the present invention will be described. Note that the present invention is not limited to the following configuration, and may be appropriately modified without departing from the gist of the present invention. The present invention also includes a combination of a plurality of preferred configurations described below.

The copper chelating agent of the present invention contains at least one selected from the group consisting of a compound represented by the following Formula (1), a compound represented by the following Formula (2), a compound represented by the following Formula (3), a compound represented by the following Formula (4), a salt thereof, and a solvate thereof.

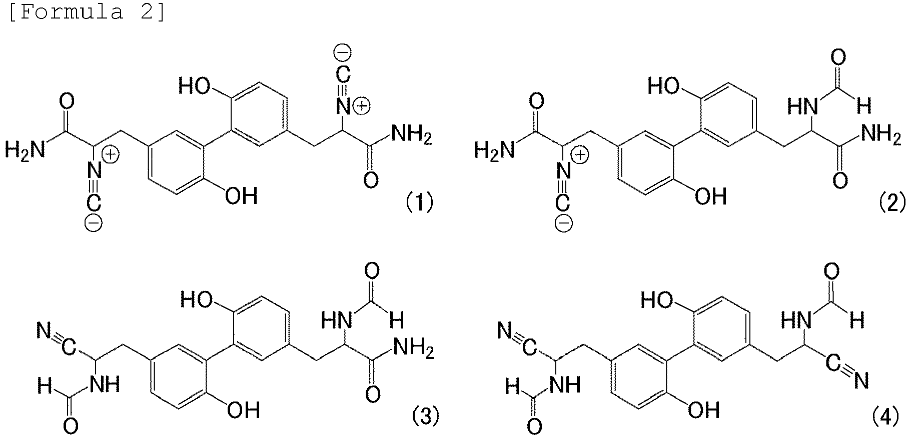

The compound represented by the above Formula (1) is also referred to as a compound (1) in the present specification. The same applies to compounds of other formula numbers, and for example, the compounds represented by Formulae (2) to (4) are also referred to as compounds (2) to (4), respectively.

At least one compound selected from the group consisting of the compound (1), the compound (2), the compound (3), the compound (4), a salt thereof, and a solvate thereof is also referred to as a compound (I). The copper chelating agent of the present invention may contain, as the compound (I), one of the above compounds or two or more thereof.

The compound (1), the compound (2), the compound (3), and the compound (4) have two asymmetric carbon atoms in the structures thereof. These compounds include any of a compound in which each asymmetric carbon atom has an R configuration, a compound in which each asymmetric carbon atom has an S configuration, and a compound of any combination thereof. In addition, any of racemic compounds, racemic mixtures, single enantiomers, and diastereomeric mixtures thereof is included in the above compounds.

The compound (1), the compound (2), the compound (3), and the compound (4) are each preferably a compound in which the absolute configurations of two asymmetric carbon atoms are both the S configuration (SS) or a compound in which the absolute configurations of two asymmetric carbon atoms are both the R configuration (RR), more preferably a compound in which the absolute configurations of two asymmetric carbon atoms are both the S configuration (SS).

Preferred embodiments of the compound represented by the above Formula (1) include a compound represented by the following Formula (1a).

Preferred embodiments of the compound represented by the above Formula (2) include a compound represented by the following Formula (2a).

Preferred embodiments of the compound represented by the above Formula (3) include a compound represented by the following Formula (3a).

Preferred embodiments of the compound represented by the above Formula (4) include a compound represented by the following Formula (4a).

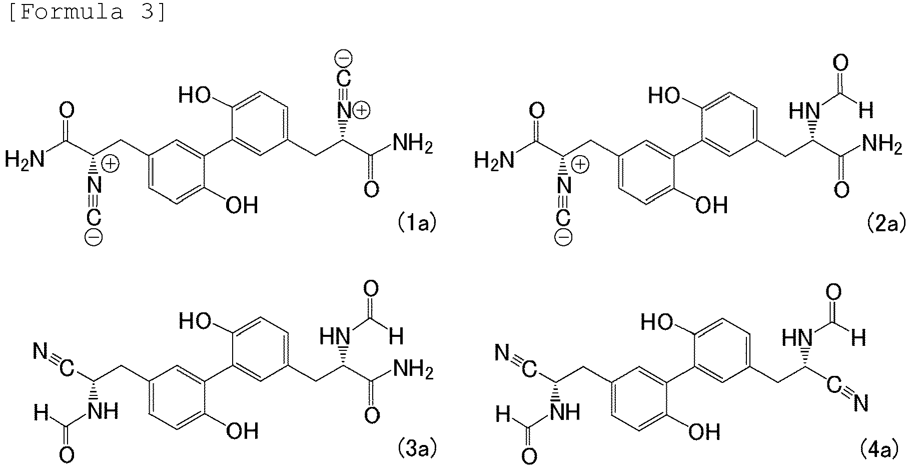

The compound (1a), the compound (2a), the compound (3a), and the compound (4a) are substances produced by actinomycetes belonging to the genus Kitasatospora. The compound (1a), the compound (2a), the compound (3a), the compound (4a), a salt thereof, and a solvate thereof are useful in various fields such as medicines and foods and beverages as substances having a copper chelating action derived from actinomycetes.

The copper chelating agent of the present invention preferably contains at least one selected from the group consisting of a compound represented by the above Formula (1), a salt thereof, and a solvate thereof. The compound (1), a salt thereof, and a solvate thereof are preferable because they have a high copper chelating action. The compound (1) is more preferably a compound represented by the above Formula (1a).

The salt of the compound is not limited as long as it is a pharmaceutically acceptable salt. As the salt, either an acidic salt or a basic salt can be employed. Examples of the acidic salt include inorganic acid salts such as hydrochloride, sulfate, nitrate, hydrofluoride, hydrobromide, and phosphate; and organic acid salts such as acetate, tartrate, lactate, citrate, fumarate, maleate, malate, succinate, methanesulfonate, ethanesulfonate, benzenesulfonate, p-toluenesulfonate, naphthalenesulfonate, and camphorsulfonate. Examples of the basic salt include alkali metal salts such as sodium salts and potassium salts; alkaline earth metal salts such as calcium salts and magnesium salts; salts with ammonia; and salts with organic amines such as morpholine, piperidine, pyrrolidine, monoalkylamine, dialkylamine, trialkylamine, mono(hydroxyalkyl)amine, di(hydroxyalkyl)amine, and tri(hydroxyalkyl)amine.

The solvate of the compound or a salt thereof is not limited as long as it is a solvate of the compound or a salt thereof, and a solvent. Examples of the solvent include water; alcohols such as ethanol and glycerol; and acetic acid. Among them, the solvate is preferably for example, a hydrate and an alcoholate, more preferably a hydrate.

The method for producing the compound (I) is not limited. The compound (I) can be produced, for example, by a known chemical synthesis method. The target compound obtained by synthesis can be purified by an ordinary purification method such as reverse phase high performance liquid chromatography, and chromatography using, for example, an ion exchange resin or a synthetic adsorbent resin.

The compound (I) can also be produced by a method including a culture step of culturing an actinomycete capable of producing the compound, and a collection step of collecting the compound or a salt thereof, or a solvate thereof from a culture obtained in the culture step.

Examples of the actinomycete capable of producing the compound (I) include actinomycetes belonging to the genus Kitasatospora, such as an actinomycete P07101 strain of the genus Kitasatospora (Non-Patent Literature 1) and a HV058 strain of Kitasatospora purpeofusca (accession number NITE BP-03475). The Kitasatospora purpeofusca HV058 strain (HV058 SIID.34879-01) has been deposited at the National Institute of Technology and Evaluation, Patent Microorganisms Depositary (292-0818, #122, 2-5-8, Kazusakamatari, Kisarazu-shi, Chiba, Japan) (accession date: 23, April, 2021, accession number: NITE BP-03475). For example, the actinomycete P07101 strain of the genus Kitasatospora described in Non-Patent Literature 1 can produce the compound (1a), the compound (2a), the compound (3a), and the compound (4a).

The culturing in the culture step can be performed according to a known method used for culturing actinomycetes.

The medium is not limited as long as the actinomycete can grow and the target substance is produced. As the medium, a known medium which is usually used as a medium for actinomycetes can be used as it is, or a medium modified as appropriate can be used. As the medium, either a liquid medium or a solid medium can be used, but a liquid medium is preferably used.

The culture conditions can also be those used for culturing actinomycetes.

By the above culturing, the compound (I) can be accumulated in the culture. The culture contains a medium and bacterial cells of actinomycetes. The compound (I) accumulates in the medium and/or in the bacterial cells of actinomycetes.

In the collection step, the compound (I) is collected from a culture obtained in the culture step. The compound (I) may be in any form of the above compound or a salt thereof, or a solvate thereof.

The method for collecting the compound (I) is not limited, and a known method used for separation and purification of a compound can be employed. The compound (I) obtained by the above method may be one type or a mixture of two or more types of compounds.

Whether the desired compound is obtained can be confirmed by a known method (for example, mass spectrometry, nuclear magnetic resonance spectroscopy (NMR), or high-performance liquid chromatography (HPLC)).

The compound represented by the above Formula (1), the compound represented by the above Formula (2), the compound represented by the above Formula (3), the compound represented by the above Formula (4), a salt thereof, and a solvate thereof have a copper chelating action. The copper chelating agent of the present invention usually contains the compound (I) as an active ingredient.

The copper chelating agent of the present invention can be used in the fields of, for example, medicines, foods and beverages, washing, and daily necessities. The copper chelating agent of the present invention can be provided in the form of, for example, medicines, foods and beverages, detergents, and daily necessities. The copper chelating agent of the present invention is usually provided as a composition containing components such as the compound (I) and an additive (also referred to as a composition for copper chelate). The copper chelating agent can contain components such as optional additives depending on its application.

For example, when the copper chelating agent of the present invention is used as a food or beverage, the food or beverage may contain, for example, any additive, component, or the like acceptable for foods or beverages, in addition to the compound or a salt thereof, or a solvate thereof.

The copper chelating agent of the present invention can be used for removal of copper ions, for example, by utilizing the copper chelating action of the compound (I). In one embodiment, the copper chelating agent of the present invention can be used, for example, in the form of a medicine, as a prophylactic or therapeutic agent for Wilson's disease, an anticancer agent, an agent for removing copper ions from the living body, and an agent for promoting excretion of copper.

In one embodiment, the copper chelating agent of the present invention can be used for removal of copper ions in the production of pharmaceutical preparations.

The copper chelating agent of the present invention can also be used for the production of foods and beverages, and the like. For example, the copper chelating agent of the present invention can be used by utilizing a copper chelating action to remove copper ions from foods and beverages. In one embodiment, copper can also be utilized by retaining copper by utilizing the copper chelating action of the compound (I). For example, it is also possible to prepare a complex in which a copper ion is coordinated to the compound (I) in advance, incorporate the complex in a health food and the like, and use the complex as a supplement of copper.

The copper chelating agent of the present invention can also be used for, for example, applications of a detergent, a stabilizer, a softener for hard water, a reagent for chelate titration of copper, a reagent for colorimetry, a copper ion buffer, a precipitant or an extractant (reagent for separation and purification of copper salt), and a reagent for gravimetry.

The compound (I) can be used for the prevention or treatment of a disease in which the copper chelating action is effective for the prevention or treatment thereof. Examples of the disease or condition in which the copper chelating action is effective for the prevention or treatment thereof include cancer and Wilson's disease.

An anticancer agent containing at least one selected from the group consisting of a compound represented by the above Formula (1), a compound represented by the above Formula (2), a compound represented by the above Formula (3), a compound represented by the above Formula (4), a salt thereof, and a solvate thereof is also one of the present invention.

A prophylactic or therapeutic agent for Wilson's disease containing at least one selected from the group consisting of a compound represented by the above Formula (1), a compound represented by the above Formula (2), a compound represented by the above Formula (3), a compound represented by the above Formula (4), a salt thereof, and a solvate thereof is also one of the present invention.

In the present specification, prevention means delaying or preventing the onset of the symptom or disease, or reducing the risk of a subject developing the symptom or disease. Treatment means alleviating the symptom or disease, preventing or delaying the worsening of the symptom or disease, or curing the symptom or disease.

The anticancer agent and the prophylactic or therapeutic agent for Wilson's disease of the present invention usually contain the compound (I) as an active ingredient.

In the anticancer agent and the prophylactic or therapeutic agent for Wilson's disease of the present invention, a preferred embodiment of the compound (I) is the same as that of the copper chelating agent described above. The anticancer agent or prophylactic or therapeutic agent for Wilson's disease of the present invention preferably contains at least one selected from the group consisting of a compound represented by the above Formula (1), a salt thereof, and a solvate thereof. The compound represented by the above Formula (1) is more preferably a compound represented by the above Formula (1a).

Hereinafter, the copper chelating agent, the anticancer agent, and the prophylactic or therapeutic agent for Wilson's disease of the present invention may be collectively referred to as "agent of the present invention".

The application subject (also referred to as an administration subject) of the agent of the present invention is not limited. Examples thereof include mammals such as human, monkey, mouse, rat, rabbit, dog, cat, pig, cow, horse, sheep, and goat, and the application subject is preferably human. Examples of the application subject of the anticancer agent include cancer patients, and subjects who desire or need prevention or treatment of cancer. Examples of the application subject of the prophylactic or therapeutic agent for Wilson's disease include patients with Wilson's disease, and subjects who desire or need prevention or treatment of Wilson's disease. Examples of the application subject of the agent of the present invention include subjects who desire or need removal of copper ions from the body, and subjects who desire or need promotion of excretion of copper ions.

The form of the agent of the present invention is not limited, and may take a form that is usually used depending on the application.

The agent of the present invention may contain optional additives in addition to the compound or a salt thereof, or a solvate thereof. For example, when the agent of the present invention is used in pharmaceutical applications, the additive is not limited as long as it is a pharmaceutically acceptable component. Examples of such additives include carriers, excipients, binders, disintegrants, lubricants, antioxidants, and colorants. The copper chelating agent, the anticancer agent, or the prophylactic or therapeutic agent for Wilson's disease of the present invention is provided, for example, as a pharmaceutical composition containing the compound (I) and an additive such as a pharmaceutically acceptable carrier.

The administration route when the copper chelating agent, the anticancer agent, or the prophylactic or therapeutic agent for Wilson's disease of the present invention is administered to a subject is not limited, and may be either oral administration or parenteral administration (for example, transdermal, transmucosal, enteral, or injection administration). Examples of the dosage form for oral administration include liquid preparations, tablets, powders, fine granules, granules, dragees, capsules, suspensions, emulsions, and chewables. Examples of the dosage form for parenteral administration include injections, drops, and external preparations for skin (for example, patches, creams, and ointments).

The content of the compound (I) in the agent of the present invention can be appropriately adjusted according to the use mode and the like. The content of the compound (I) in the copper chelating agent, the anticancer agent, or the prophylactic or therapeutic agent for Wilson's disease may be, for example, 0.0001 wt% or more and 90 wt% or less, preferably 0.001 wt% or more and 50 wt% or less. When two or more types of compounds are used for the compound (I), the amount of the compound (I) is the total thereof.

When the compound (I) is administered to a subject, the dose thereof can be appropriately set according to, for example, the administration route, the application subject, the body weight of the subject, and the disease, but is not limited thereto. For example, when the compound (I) is administered to an adult, the compound (I) can be administered in a range of 0.001 mg/kg or more and 100 mg/kg or less per day. The number of administrations is, for example, once, twice, or three times per day.

### [Methods of prevention or treatment]

The present invention also encompasses a method for preventing or treating cancer or Wilson's disease by administering the compound (I) described above. The present invention also encompasses use of the compound (I) for the prevention or treatment of cancer or Wilson's disease.

The present invention also encompasses use of the compound (I) for the production of a copper chelating agent. The present invention also encompasses use of the compound (I) for the production of an anticancer agent or a prophylactic or therapeutic agent for Wilson's disease.

The present invention also encompasses a method for promoting excretion of copper ions, or a method for removing copper ions by administering the compound (I) described above. The present invention also encompasses use of the compound (I) for producing an agent for removing copper ions from the living body, and an agent for promoting excretion of copper ions.

In the above method and use, a preferred subject and the like are the same as the preferred application subject (administration subject) and the like of the agent of the present invention.

### EXAMPLES

Hereinafter, examples more specifically disclosing the present invention will be described. Note that the present invention is not limited only to these examples.

### [Preparation Example 1]

The compound (1a) and the compound (2a) were obtained by culturing a Kitasatospora purpeofusca HV058 strain (hereinafter, described as HV058 strain).

### (Culturing of HV058 strain)

A Kitasatospora purpeofusca HV058 strain was seeded on the following medium and cultured. The Kitasatospora purpeofusca HV058 strain has been deposited at the National Institute of Technology and Evaluation, Patent Microorganisms Depositary (292-0818, #122, 2-5-8, Kazusakamatari, Kisarazu-shi, Chiba, Japan) (accession date: 23, April, 2021, accession number: NITE BP-03475).
Medium specification: 1 L of ISP medium 4 to which 4% DIAION (registered trademark) HP20 (manufactured by Mitsubishi Chemical Corporation) was added
Culture conditions: 30°C, 7 days, 140 rpm
ISP medium 4 was prepared and used in the following composition.
Components (per 1 L): Soluble starch (10.000 g), K₂HPO₄ (1.000 g), MgSO₄·7H₂O (1.000 g), NaCl (1.000 g), (NH₄)₂SO₄ (2.000 g), CaCO₃ (2.000 g), FeSO₄·7H₂O (0.001 g), MnCl₂·7H₂O (0.001 g), ZnSO₄·7H₂O (0.001 g)
Final pH (25°C): 7.2 ± 0.2

### (Purification of compound)

After the above culturing, the fraction containing the bacterial cells and the synthetic adsorbent was separated by centrifugation from the culture solution of the HV058 strain to which the synthetic adsorbent was added. Then, the bacterial cells and DIAION HP20 were washed with water in an amount 5 times the volume thereof. Subsequently, to the bacterial cells and DIAION HP20, methanol in an amount of 5 times the volume thereof was added, followed by extraction to obtain a crude extraction fraction. The obtained crude extraction fraction was fractionated by silica gel column chromatography. Specifically, the crude extraction fraction was subjected to silica gel column chromatography (Silica gel 60N (spherical, neutral) 63 to 210 µm, manufactured by Kanto Chemical Co., Inc.), and elution was performed while the methanol content was increased stepwise by 10% from 90% chloroform +10% methanol to finally 100% methanol. The desired compound was detected in a 90% chloroform +10% methanol fraction (referred to as a fraction (I)) and a 70% chloroform +30% methanol fraction (referred to as a fraction (II)). Thus, the desired product was purified from these fractions (I) and (II) by reverse phase high performance liquid chromatography.

The fraction (I) obtained above was vacuum-dried, and then the obtained powder was dissolved in methanol and purified under the following conditions by high-performance liquid chromatography (HPLC) to fractionate the water-soluble fraction.
Apparatus: High-performance liquid chromatograph system Prominence (manufactured by Shimadzu Corporation)
System controller: CBM-20 Alite
Pump: LC-20AD
Detector: SPD-M20A
Column: Cosmosil 5C₁₈MS-II 10 mm I.D. × 250 mm (manufactured by Nacalai Tesque, Inc.)

Elution conditions:
Liquid A: H₂O
Liquid B: Acetonitrile
Concentration of liquid B: 15 vol%
Flow rate: 3.0 mL/min
Temperature: 30°C
Detection: 210 nm

Target peaks were detected at elution times of 12 minutes, 25 minutes, and 35 minutes under the above conditions.

As a result of analyzing fragment ions of the peak at an elution time of 12 minutes by LC-MS/MS, it was found that a compound represented by the following Formula (3a) (compound (3a)) was obtained.

As a result of analyzing fragment ions of the peak at an elution time of 25 minutes by LC-MS/MS, it was found that a compound represented by the following Formula (1a) (compound (1a)) was obtained.

As a result of analyzing fragment ions of the peak at an elution time of 35 minutes by LC-MS/MS, it was found that a compound represented by the following Formula (4a) (compound (4a)) was obtained.

The fraction (II) obtained above was vacuum-dried, and then the resulting powder was dissolved in methanol and purified under the above conditions by HPLC in the same manner as the fraction (I) to fractionate the water-soluble fraction.

As a result, a target peak was detected at an elution time of 20 minutes. As a result of analyzing fragment ions by LC-MS/MS, it was found that a compound represented by the following Formula (2a) (compound (2a)) was obtained.

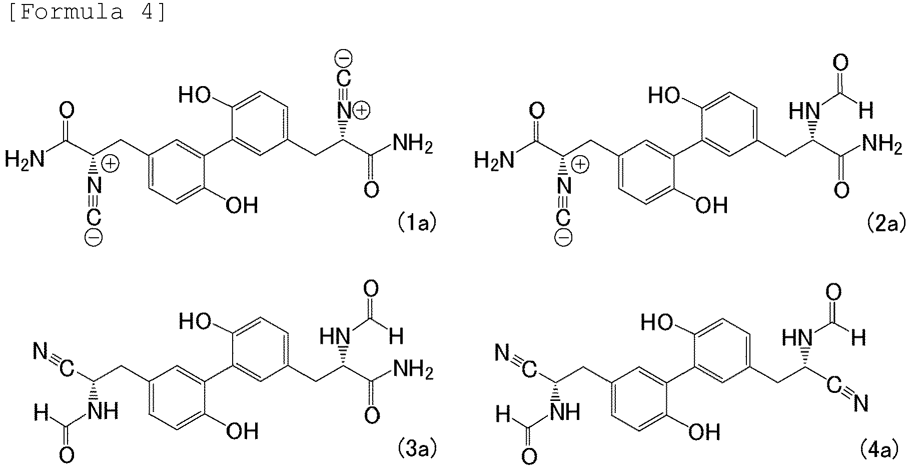

### [Example 1]

The copper chelating action of the compound represented by the above Formula (1a) (compound (1a)) and the compound represented by the above Formula (2) (compound (2a)) was evaluated by the following method.

### (Method for evaluating copper chelating action)

An aqueous CuCl₂ solution was added to a methanol solution of 10 mM compound (1a) or 10 mM compound (2a) so as to have a final concentration of 100 mM, and the mixture was shaken at 30°C for 60 minutes, and then analyzed by LC-MS. A methanol solution of 10 mM compound (1a) or 10 mM compound (2a) to which an aqueous CuCl₂ solution was not added was also analyzed by LC-MS.

### (LC-MS measurement conditions)

Pure water to which 0.1% formic acid was added was used as a solvent A, and acetonitrile was used as a solvent B. The samples were separated on a reversed-phase column (Cosmosil 5.0C₁₈-MS-II 2.0 × 100 mm (manufactured by Nacalai Tesque, Inc.)). At this time, the flow rate was set to 0.2 ml/min, and elution was performed in a gradient mode in which the proportion of the solvent B was set to 2% until 2 minutes, and then changed from 2 to 98% from 2 to 15 minutes. The eluted sample was ionized by electrospray ionization method in a positive mode.

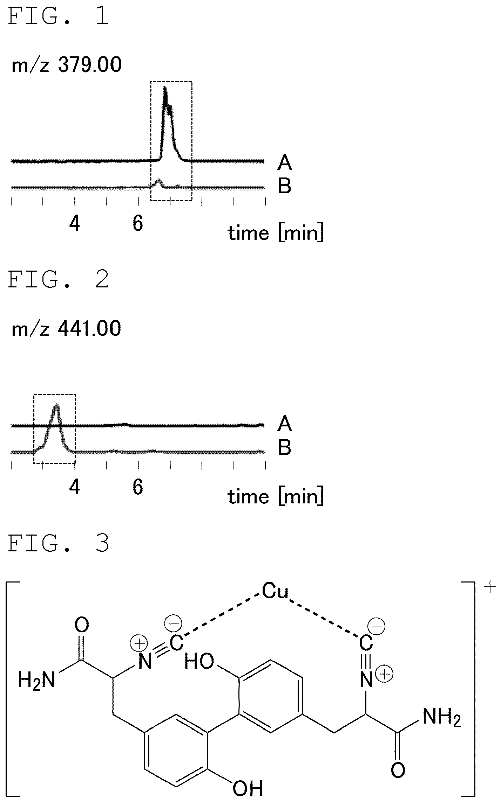

The results of the LC-MS analysis are shown in FIGS. 1, 2, 4 and 5.

FIGS. 1 and 2 show the results of LC-MS analysis of a compound (1a) and a sample in which a copper ion is added to the compound (1a). FIG. 1 is an MS spectrum of ions at m/z 379.00. FIG. 2 is an MS spectrum of ions at m/z 441.00. In FIGS. 1 and 2, A is a compound (1a) (a methanol solution of the compound (1a) to which a copper ion is not added), and B is a sample in which a copper ion is added to the compound (1a) (a sample in which a copper ion is added to a methanol solution of the compound (1a)).

The ion at m/z 379.00 is an ion of the compound (1a). From FIG. 1, when a copper ion was not added to the compound (1a), a peak of the ion at m/z 379.00 (peak surrounded by a dotted line) was observed (A in FIG. 1). Meanwhile, in the sample in which a copper ion was added to the compound (1a), the peak of the ion at m/z 379.00 was not observed (B in FIG. 1).

The ion at m/z 441.00 is an ion of a complex of the compound (1a) and a copper ion. From FIG. 2, in the sample in which a copper ion was added to the compound (1a), a peak of the ion at m/z 441.00 (peak surrounded by a dotted line) was observed (B in FIG. 2). Meanwhile, when a copper ion was not added to the compound (1a), the peak of the ion at m/z 441.00 was not observed (A in FIG. 2).

The above results show that the compound (1a) has a copper chelating action. FIG. 3 shows an estimated chemical formula of a complex of a compound (1a) and a copper ion.

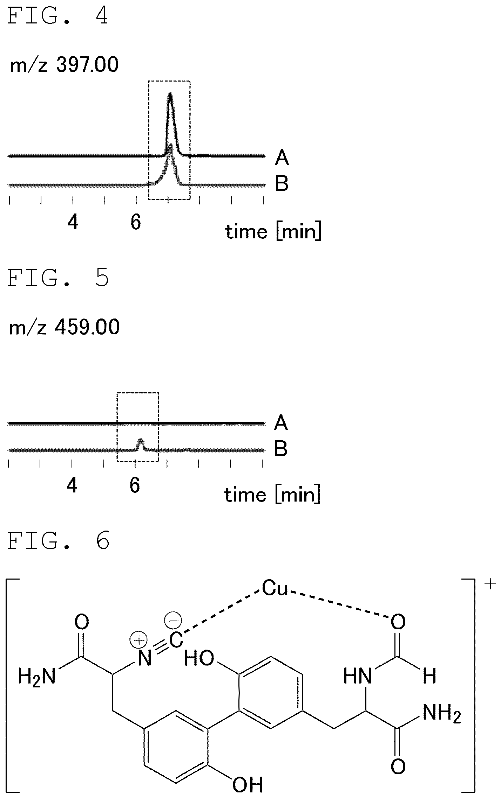

FIGS. 4 and 5 show the results of LC-MS analysis of a compound (2a) and a sample in which a copper ion is added to the compound (2a). FIG. 4 is an MS spectrum of ions at m/z 397.00. FIG. 5 is an MS spectrum of ions at m/z 459.00. In FIGS. 4 and 5, A represents a compound (2a) (a methanol solution of the compound (2a) to which a copper ion is not added), and B represents a sample in which a copper ion is added to the compound (2a) (a sample in which a copper ion is added to a methanol solution of the compound (2a)).

The ion at m/z 397.00 is an ion of the compound (2a). From FIG. 4, when a copper ion was not added to the compound (2a), a peak of the ion at m/z 397.00 (peak surrounded by a dotted line) was observed (A in FIG. 4). Meanwhile, in the sample in which a copper ion was added to the compound (2a), the peak of the ion at m/z 397.00 was small (B in FIG. 4).

The ion at m/z 459.00 is an ion of a complex of the compound (2a) and a copper ion. From FIG. 5, in the sample in which a copper ion was added to the compound (2a), a peak of the ion at m/z 459.00 (peak surrounded by a dotted line) was observed (B in FIG. 5). Meanwhile, when a copper ion was not added to the compound (2a), the peak of the ion at m/z 459.00 was not observed (A in FIG. 5).

The above results show that the compound (2a) has a copper chelating action. FIG. 6 shows an estimated chemical formula of a complex of a compound represented by Formula (2a) and a copper ion.

A comparison between FIGS. 1 and 2 and FIGS. 4 and 5 show that the copper chelating action of the compound (1a) is stronger than that of the compound (2a). From these results, it was presumed that the isonitrile group (-NC) of these compounds exhibited a copper chelating action.

## Claims

1. A copper chelating agent comprising at least one selected from the group consisting of a compound represented by the following Formula (1), a compound represented by the following Formula (2), a compound represented by the following Formula (3), a compound represented by the following Formula (4), a salt thereof, and a solvate thereof.

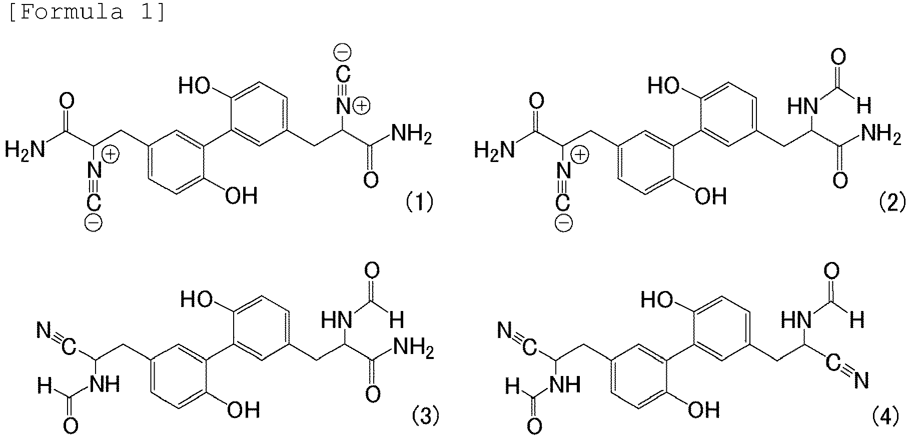

2. An anticancer agent comprising at least one selected from the group consisting of a compound represented by the following Formula (1), a compound represented by the following Formula (2), a compound represented by the following Formula (3), a compound represented by the following Formula (4), a salt thereof, and a solvate thereof.

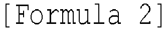

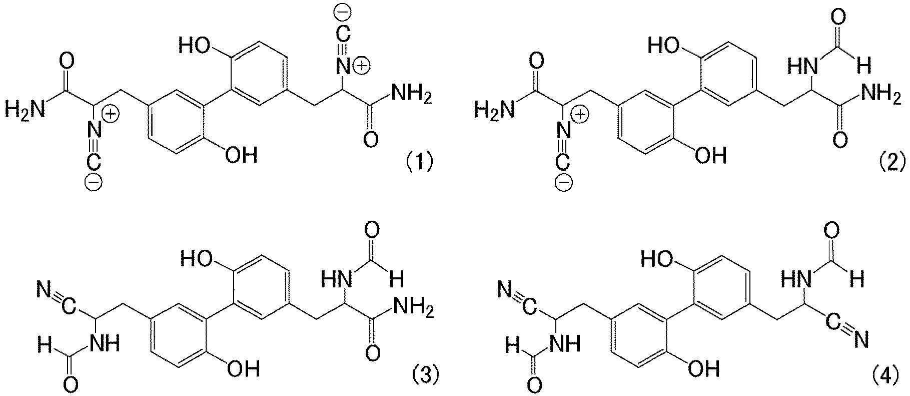

3. A prophylactic or therapeutic agent for Wilson's disease, comprising at least one selected from the group consisting of a compound represented by the following Formula (1), a compound represented by the following Formula (2), a compound represented by the following Formula (3), a compound represented by the following Formula (4), a salt thereof, and a solvate thereof.

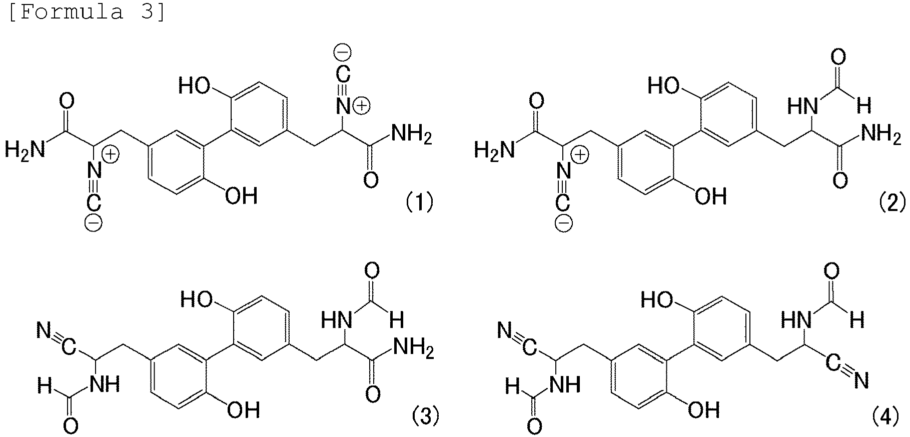

4. The agent according to any one of claims 1 to 3, comprising at least one selected from the group consisting of the compound represented by Formula (1), a salt thereof, and a solvate thereof.
